(19) Europäisches Patentamt
European Patent Office
Office européen des brevets

(11) Veröffentlichungsnummer: **0 167 821**
**A1**

# (12) EUROPÄISCHE PATENTANMELDUNG

(21) Anmeldenummer: 85106909.6

(22) Anmeldetag: 04.06.85

(51) Int. Cl.⁴: C 07 D 209/92

(30) Priorität: 14.06.84 DE 3422075

(43) Veröffentlichungstag der Anmeldung: 15.01.86 Patentblatt 86/3

(84) Benannte Vertragsstaaten: CH DE FR GB IT LI

(71) Anmelder: BAYER AG
Konzernverwaltung RP Patentabteilung
D-5090 Leverkusen 1 Bayerwerk(DE)

(72) Erfinder: Marzolph, Gerhard, Dr.
Semmelweisstrasse 87a
D-5000 Köln 80(DE)

(72) Erfinder: Blank, Heinz Ulrich, Dr.
Am Geusfelde 35
D-5068 Odenthal(DE)

## (54) Verfahren zur Durchführung von Friedel-Crafts-Reaktionen und Friedel-Crafts-ähnlichen Reaktionen.

(57) Friedel-Crafts- und Friedel-Crafts-ähnliche Reaktionen, die in inerten wasserfreien Lösungsmitteln und in Gegenwart von wasserfreien Halogeniden des Aluminiums, Eisens oder Zinks durchgeführt werden, lassen sich so weiterführen, daß man nach Beendigung der Reaktion dem Reaktionsgemisch Halogenide der Alkali- oder Erdalkalimetalle oder des gegebenenfalls substituierten Ammoniums bei 20-250°C zusetzt und gegebenenfalls nach einer Nachreaktionszeit das Lösungsmittel mindestens teilweise in wasserfreier Form durch Destillation aus dem Reaktionsgemisch zurückgewinnt.

Das erfindungsgemäße Verfahren eignet sich unter anderem zur Cyclisierung von gegebenenfalls substituiertem alpha-Naphtylisocyanat der Formel (I) zum gegebenenfalls substituierten Benz-[c,d]-indol-2-on (Naphtostyril) der Formel (II)

EP 0 167 821 A1

BAYER AKTIENGESELLSCHAFT 5090 Leverkusen, Bayerwerk

Konzernverwaltung RP Ha/by-c 13.06.84
Patentabteilung

# Verfahren zur Durchführung von Friedel-Crafts-Reaktionen und Friedel-Crafts-ähnlichen Reaktionen

Die vorliegende Erfindung betrifft ein Verfahren zur Durchführung von Friedel-Crafts-Reaktionen und Friedel-Crafts-ähnlichen Reaktionen in inerten wasserfreien Lösungsmitteln und in Gegenwart von wasserfreien Halogeniden des Aluminiums, Eisens oder Zinks, bei dem nach Beendigung der Reaktion dem Reaktionsgemisch Halogenide der Alkali- oder Erdalkalimetalle oder des gegebenenfalls substituierten Ammoniums zugesetzt werden. Insbesondere betrifft die Erfindung ein Verfahren zur Herstellung von gegebenenfalls substituierten Benz-[c,d]-indol-2-on (Naphthostyril) durch Umsetzung von gegebenenfalls substituiertem α-Naphthylisocyanat in einem inerten wasserfreien Lösungsmittel in Gegenwart von wasserfreien Halogeniden des Aluminiums, Eisens oder Zinks.

Es ist bekannt, Friedel-Crafts-Reaktionen und Friedel-Crafts-ähnliche Reaktionen in inerten wasserfreien Lösungsmitteln und in Gegenwart von wasserfreien Halogeniden des Aluminiums, Eisens oder Zinks durchzuführen

(Olah, Friedel-Crafts and Related Reactions, Band 1-4, Interscience, 1963).

Beispiele für solche Reaktionen sind: Alkylierungen von aromatischen Verbindungen mit aliphatischen Halogeniden, Olefinen, Alkoholen oder Estern; Acylierungen von aromatischen Verbindungen mit Säurehalogeniden oder Säureanhydriden, wie z.B. die Umsetzung von Chlorbenzol mit Acetylchlorid zu p-Choracetophenon; Acylierungen von aromatischen Verbindungen mit aliphatischen oder aromatischen Isocyanaten und N,N-disubstituierten Carbamidsäurehalogeniden oder N,N-disubstituierten Carbamidsäureestern, wie z.B. die Umsetzung von α-Naphthylisocyanat zum Benz-[c,d]-indol-2-on (Naphthostyril) oder von N-Ethyl-N-α-naphthylcarbamidsäurechlorid oder von N-Ethyl-N-α-naphthylcarbamidsäurephenylester zum N-Ethyl-benz-[c,d]-indol-2-on (Ethylnaphtholactam).

Beispiele für Friedel-Crafts-ähnliche Reaktionen sind: die Halogenierung von aromatischen Verbindungen mit Chlor, Brom oder Gemischen aus Chlor und Brom in Gegenwart von Friedel-Crafts-Katalysatoren, wie z.B. die Bromierung von Benzaldehyd zum 3-Brombenzaldehyd oder von Acetophenon zum 3-Bromacetophenon; die Isomerisierung der Halogenbenzole an $AlCl_3$ bei erhöhter Temperatur, wie z.B. die Umsetzung von o-Dichlorbenzol und p-Dichlorbenzol zu m-Dichlorbenzol; und ebenso die Isomerisierung von Alkylbenzolen, wie z.B. die Umsetzung von 2,4-Dichlor-isopropylbenzol zu 3,5-Dichlor-isopropylbenzol.

Als Beispiel für eine Friedel-Crafts-ähnliche Reaktion soll auf die Herstellung von Naphthostyril eingegangen werden, das ein bekanntes Zwischenprodukt für wertvolle Farbstoffe ist (GB 973 259). Hierbei werden exemplarische Nachteile der bisherigen Durchführung von Friedel-Crafts- und Friedel-Crafts-ähnlichen Reaktionen deutlich.

So ist es aus US 2.628.964 bekannt, daß α-Naphthylisocyanat mit $AlCl_3$ zu Naphthostyril umgesetzt werden kann. Hierzu wird α-Naphthylisocyanat in etwa 4,5 gew.-%iger Lösung, beispielsweise in Dichlorbenzol, mit 2,5 Mol $AlCl_3$ pro Mol Isocyanat bei 160°C cyclisiert; anschließend wird mit wäßriger Salzsäure der Friedel-Crafts-Komplex des $AlCl_3$ hydrolysiert und das Lösungsmittel durch Wasserdampfdestillation ausgetrieben. Das Rohprodukt wird zunächst isoliert, mit wäßriger Natronlauge gelöst, mit Salzsäure gefällt und dann durch Filtration und Trocknung isoliert. Dieses Verfahren besitzt als schwerwiegende Nachteile das Arbeiten in stark verdünnter Lösung bei der Cyclisierung, das zu schlechten Raum-Ausbeuten führt, und die Wasserdampfdestillation der großen Lösungsmittelmengen, die sehr lange dauert und energieaufwendig ist.

In der DE-OS 27 00 649 werden diese Probleme durch konzentriertere Arbeitweise im Reaktionsgemisch, beispielsweise bis zu 15 Gew.-% Naphthylisocyanat in der Lösung und durch nachgeschaltete Phasentrennung statt der Wasserdampfdestillation zunächst umgangen.

Es zeigte sich jedoch, daß die Cyclisierungsreaktion in dieser konzentrierten Lösung nur in einem sehr engen Temperaturintervall zu befriedigenden Ausbeuten führt. Das Einhalten der engen Temperaturgrenzen erfordert aber bei der technischen Durchführung eine aufwendige und teure Regelung.

Außerdem ist die Phasentrennung mit Problemen verbunden, wie das Beispiel 1 der genannten DE-OS zeigt: Hier wird nach der Hydrolyse zunächst ein Teil der organischen Phase abgehebert, dann Isopropanol zugesetzt, ein Teil der Wasserphase abgelassen, erneut Wasser zugesetzt, und nun werden endgültig die Phasen getrennt. Im Beispiel 39 dieser DE-OS muß die Hydrolysemischung 5 Stunden gerührt werden, bevor die Phasentrennung durchgeführt werden kann. In Beispiel 40 wird soviel Wasser zugegeben, daß die Isolierung aus einer etwa 2,5 %igen Suspension erfolgt. Damit ist aber der Vorteil der konzentrierten Arbeitsweise bei der eigentlichen Cyclisierung durch die Schwierigkeit der Aufarbeitung zunichte gemacht.

In allen genannten Arbeitsvarianten fällt das Lösungsmittel wasserfeucht an und muß vor der Wiederverarbeitung getrocknet und erneut destilliert werden.

Es wurde nun ein Verfahren zur Durchführung von Friedel-Crafts-Reaktionen und Friedel-Crafts-ähnlichen Reaktionen in inerten wasserfreien Lösungsmitteln und in Gegenwart von wasserfreien Halogeniden des Aluminiums, Eisens oder

Zinks gefunden, das dadurch gekennzeichnet ist, daß man nach Beendigung der Reaktion dem Reaktionsgemisch Halogenide der Alkali- oder Erdalkalimetalle oder des gegebenenfalls substituierten Ammoniums in einer Menge von 100-150 % der zur Bildung der Tetrahalogenosalze des Aluminiums, Eisens oder Zinks erforderlichen Menge bei 20-250°C zusetzt und gegebenenfalls nach einer Nachreaktionszeit das Lösungsmittel mindestens teilweise in wasserfreier Form durch Destillation aus dem Reaktionsgemisch zurückgewinnt.

Das erfindungsgemäße Verfahren bezieht sich auf Friedel-Crafts-Reaktionsgemische die unter Verwendung von wasserfreien Halogeniden des Aluminiums, Eisens oder Zinks, bevorzugt des Aluminiums oder Eisens, besonders bevorzugt des Aluminiums, erhalten werden. Als Halogenide kommen hierbei die Chloride, Bromide oder Iodide, bevorzugt die Chloride oder Bromide in Frage. Insbesondere seien $AlCl_3$ oder $AlBr_3$ oder ein Gemisch daraus genannt. Die beschriebenen Stoffe werden im allgemeinen als Friedel-Crafts-Katalysatoren bezeichnet. Die Menge solcher Katalysatoren variiert sehr stark. So ist beispielsweise bei der Alkylierung von Aromaten unter Verwendung von Alkylhalogeniden oder Olefinen eine katalytische Menge des Katalysators ausreichend, während bei der Alkylierung von Aromaten mit Alkoholen oder bei Acylierungsreaktionen mindestens stöchiometrische Mengen des Katalysators erforderlich sind. Als mindestens stöchiometrische Mengen seien beispielsweise 1-4, bevorzugt 1,2-3, besonders bevorzugt 1,5-2,5 Mol Katalysator pro Mol Substrat genannt. Der Katalysator kann in bekannter Weise in fester Form zum Reaktionsgemisch gegeben werden oder als 10-30 gew.-%ige Lösung in einem geeigneten Lösungsmittel zugesetzt oder vorgelegt werden.

Als inerte Lösungsmittel, die in wasserfreier Form benutzt werden, kommen alle diejenigen in Frage, die unter den Reaktionsbedingungen mit dem Katalysator oder den Substraten nicht reagieren. Beispielhaft aus der dem Fachmann bekannten großen Anzahl solcher Lösungsmittel sei Chlorbenzol, o-Dichlorbenzol, Dichlorbenzol-Isomerengemische, Trichlorbenzol oder Chlortoluole genannt.

Die Friedel-Crafts- oder Friedel-Crafts-ähnliche Reaktion kann drucklos und/oder unter erhöhtem Druck durchgeführt werden, wobei die Variante unter erhöhtem Druck vornehmlich beim Einsatz von flüchtigen Olefinen oder Alkylhalogeniden angewandt wird. Die Temperatur für solche Reaktionen schwankt in einem außerordentlich großen Maß. Bei Alkylierungen wählt man häufig tiefe Temperaturen bis etwa Raumtemperatur, während Acylierungsreaktionen und Cyclisierungsreaktionen ohne Spaltprodukte häufig bei Temperaturen bis hinauf zu 200°C durchgeführt werden. Das Ende der Reaktion kann beispielsweise dadurch erkannt werden, daß die Abspaltung von Halogenwasserstoff aufhört. Bei Cyclisierungsreaktionen ohne Spaltprodukte kann das Ende der Reaktion durch geeignete analytische Methoden, beispielsweise durch Verfolgung des Reaktionsansatzes mit Hilfe der Hochdruckflüssigkeitschromatographie(HFC)-Analyse verfolgt werden.

Nach Beendigung der Reaktion werden dem Reaktionsgemisch Halogenide der Alkali- oder Erdalkalimetalle oder des gegebenenfalls substituierten Ammoniums, also Salze, zugesetzt. Als Kationen für diese Salze seien beispielsweise genannt: Lithium, Natrium, Kalium, Magnesium, Calcium, Ammonium oder durch offenkettige oder cyclische, geradkettige oder verzweigte Alkylgruppen, Arylgruppen, Aralkylgruppen oder heterocyclische Gruppen einfach, zweifach, dreifach oder vierfach substituiertes Ammonium genannt. Bevorzugtes Kation ist Natrium, Kalium, Calcium, Ammonium oder die genannten ein- bis vierfach substituierten Ammoniumionen. In besonders bevorzugter Weise seien Natrium und Kalium als Kationen solcher Salze genannt. Als Anionen solcher Salze kommen in Frage die Fluoride, Chloride, Bromide oder Iodide, bevorzugt die Chloride oder Bromide. Solche Salze können einzeln oder im Gemisch angewandt werden. Diese Salze werden erfindungsgemäß in wasserfreier Form eingesetzt.

Die Salze werden in einer Menge von 100-150 %, bevorzugt 100 bis 120 % der zur Bildung der Tetrahalogenosalze des Aluminiums, Eisens oder Zinks erforderlichen Menge eingesetzt. Der Einsatz einer noch größeren Menge bringt keinen weiteren Vorteil.

Typische erfindungsgemäß einsetzbare Salze sind NaCl, KCl, $CaCl_2$, NaBr, KBr, $CaBr_2$, $NH_4Cl$ und $NH_4Br$.

Die Salze werden dem Reaktionsgemisch bei einer Temperatur von 20-250°C, bevorzugt 50-200°C, besonders bevorzugt 100-180°C, zugesetzt. Danach kann sich eine Nachreaktionszeit, beispielsweise 0-3 Stunden, bevorzugt 0,25-1 Stunde anschließen. Hierbei wird man im allgemeinen eine längere Nachreaktionszeit ansetzen, falls die Temperatur der Salzzugabe und der Nachreaktion tiefer liegt und umgekehrt. Anschließend wird das Lösungsmittel aus dem Reaktionsgemisch durch Destillation in einer dem Fachmann grundsätzlich bekannten Weise zurückgewonnen, wobei bei Normaldruck oder bei vermindertem Druck gearbeitet werden kann. Hierbei wird das Lösungsmittel in einer wasserfreien, unmittelbar wiederverwendbaren Form gewonnen. In dieser Form werden mindestens 80 % des eingesetzten Lösungsmittels, vielfach jedoch über 90 % zurückgewonnen. Unter günstigen Umständen ist es sogar möglich, das gesamte Lösungsmittel vom Reaktionsgemisch destillativ in der beschriebenen sauberen Form zu gewinnen und darüber hinaus anschließend das durch die Friedel-Crafts- (oder -ähnliche) Reaktion gewonnene Produkt destillativ zu gewinnen, sofern dieses Produkt, gegebenenfalls unter vermindertem Druck, unzersetzt destillierbar ist.

Zur weiteren Aufarbeitung wird der Rückstand der beschriebenen Lösungsmittel-Wiedergewinnung mit gegebenenfalls angesäuertem Wasser versetzt. Für den Fall, daß das Lösungsmittel nicht vollständig destillativ abgetrennt worden war, wird nunmehr in geeigneter und dem Fachmann bekannter Weise eine Trennung der organischen und der wäßrigen Phase vorgenommen. Die wäßrige Phase enthält die anorganischen Bestandteile und wird gegebenenfalls nochmals mit organischem Lösungsmittel extrahiert. Dieser Extrakt wird mit der organischen Phase vereinigt und in bekannter Weise, gegebenenfalls nach vorangegangener Trocknung, auf restliches Lösungsmittel und das Friedel-Crafts-Produkt aufgearbeitet.

Für den Fall, daß das gesamte Lösungsmittel destillativ wiedergewonnen wurde, enthält der Rückstand die anorganischen Bestandteile und das Friedel-Crafts-Produkt. Für den Fall, daß ein flüssiges Friedel-Crafts-Produkt vorliegt, kann dies beispielsweise durch bloße Filtration von den anorganischen Bestandteilen getrennt werden. Es kann jedoch auch in einem geeigneten Lösungsmittel aufgenommen werden, danach durch Filtration von den anorganischen Bestandteilen getrennt werden und dann durch Kristallisation oder fraktionierte Destillation weiter aufgearbeitet werden.

Für den Fall, daß sowohl das Lösungsmittel als auch das Friedel-Crafts-Produkt destillativ aus dem nach der erfindungsgemäßen Durchführung erhaltenen Reaktionsgemisch gewonnen worden waren, enthält der Rückstand die anorganischen Bestandteile und gegebenenfalls noch geringe Mengen des Friedel-Crafts-Produkts. Dieser Rückstand wird dann gegebenenfalls mit einem geeigneten Lösungsmittel extrahiert, um solche Reste von Friedel-Crafts-Produkt zu gewinnen. Extrakt und

Rückstand werden dann in der beschriebenen Weise weiterbehandelt.

Das bei der erfindungsgemäßen Lösungsmittel-Wiedergewinnung anfallende Lösungsmittel hat eine hohe Reinheit, ist wasserfrei und kann daher direkt in den nächsten Friedel-Crafts-Reaktionsansatz eingesetzt werden. Bei der Verwendung von Aluminiumhalogeniden als Friedel-Crafts-Katalysator enthält ein solches wiedergewonnenes Lösungsmittel beispielsweise weniger als 20 ppm Aluminium. Die erfindungsgemäße Destillation des Lösungsmittels in Gegenwart der oben beschriebenen Metallhalogenide ist raumsparend und zeitsparend, weil nicht in Gegenwart einer zusätzlichen Wasserphase wie bei der Wasserdampfdestillation gearbeitet werden muß. Wollte man im Gegensatz zur erfindungsgemäßen Arbeitsweise aus einem Friedel-Crafts-Reaktionsansatz nach herkömmlicher Technik das Lösungsmittel abdestillieren, um eine umständliche Wasserdampfdestillation zu sparen, so wäre mit einer erheblichen Sublimation des verwendeten Friedel-Crafts-Katalysators zu rechnen. Diese Sublimation setzt sich bis in die Lösungsmittelvorlage fort. Daher kann es an engen Stellen zu Verstopfungen kommen, zu deren Beseitigung das Verfahren unterbrochen und die Destillationsapparatur zerlegt werden muß. Außerdem enthielte ein solches Destillat beträchtliche Mengen des verwendeten Friedel-Crafts-Katalysators, der vor einer Zwischenlagerung des Lösungsmittels durch Filtrieren, Waschen mit Wasser und erneuter Destillation entfernt werden müßte.

Auch eine direkte Wiederverwendung eines solchen durch Friedel-Crafts-Katalysator kontaminierten Lösungsmittels im nächsten Reaktionsansatz wäre erschwert, weil ein solches Lösungsmittel durch den Gehalt an Katalysator verstärkt hygroskopisch wäre und weil weiterhin der Gehalt an Katalysator getrennt bestimmt und berücksichtigt werden müßte.

Das erfindungsgemäße Verfahren eignet sich unter anderem in hervorragender Weise zur Herstellung von Benz-/c,d/-indol-2-on (Naphthostyril) aus α-Naphthylisocyanat. Die Erfindung betrifft daher insbesondere ein Verfahren zur Herstellung von gegebenenfalls substituiertem Naphthostyril durch Umsetzung von gegebenenfalls substituiertem α-Naphthylisocyanat in einem inerten wasserfreien Lösungsmittel in Gegenwart von wasserfreien Halogeniden des Aluminiums, Eisens oder Zinks, das dadurch gekennzeichnet ist, daß man nach Beendigung der Reaktion in der oben beschriebenen Weise weiterverfährt.

Als gegebenenfalls substituiertes α-Naphthylisocyanat sei beispielsweise ein solches der Formel

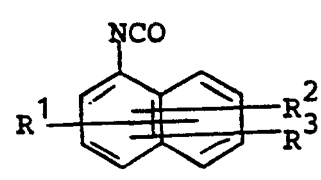

(I)

genannt, in der

$R^1$, $R^2$ und $R^3$ unabhängig voneinander für Wasserstoff, gegebenenfalls substituiertes geradkettiges oder

verzweigtes Alkyl, Cycloalkyl, Aryl oder Aralkyl, Halogen, Cyano, Alkoxycarbonyl, Alkylcarbonyloxy, Aryloxycarbonyl, Arylcarbonyloxy, Alkylcarbonyl, Arylcarbonyl, Alkoxy, Aryloxy, Acylamino, Dialkylamino, Alkylarylamino, Alkylsulfonyl oder Arylsulfonyl stehen, wobei weiterhin zwei der Reste $R^1$, $R^2$ und $R^3$ gemeinsam einen anellierten aromatischen oder cycloaliphatischen Ring darstellen können.

Als Alkyl sei beispielsweise solches mit 1-20, bevorzugt 1-10, besonders bevorzugt 1-4 Kohlenstoffatomen genannt, wie Methyl, Ethyl, Propyl, Butyl, Hexyl, Decyl, Hexadecyl oder Eicosyl.

Als Cycloalkyl sei beispielsweise solches mit 4-8, bevorzugt 5-6 im Ring angeordneten Kohlenstoffatomen genannt, das gegebenenfalls eine oder zwei Methyl- oder Ethylgruppen tragen kann, wie Cyclobutyl, Cyclopentyl, Cyclohexyl, Cycloheptyl, Cyclooctyl, Methyl-cyclopentyl, Ethyl-cyclopentyl, Methyl-cyclohexyl und Ethyl-cyclohexyl.

Als Aryl sei beispielsweise Phenyl, Naphthyl, Anthryl oder Biphenyl, bevorzugt Phenyl, genannt.

Als Aralkyl sei beispielsweise Benzyl, Phenylethyl, Naphthyl-methyl, Naphthyl-ethyl oder Anthrylmethyl, bevorzugt Benzyl, genannt.

Die genannten Substituenten, insbesondere die aromatischen Teile, können durch Halogen, Cyano, Alkoxy, Aryloxy, Acylamino, Dialkylamino oder $C_1$-$C_4$-Alkyl substituiert sein.

Als Halogen sei beispielsweise Fluor, Chlor, Brom, Iod, bevorzugt Fluor, Chlor, Brom, besonders bevorzugt Chlor oder Brom, genannt.

In bevorzugter Weise bedeuten die Substituenten $R^1$, $R^2$ und $R^3$ unabhängig voneinander Wasserstoff, Halogen, $C_1$-$C_4$-Alkyl, $C_1$-$C_4$-Alkoxy, CO-NH-$C_1$-$C_4$-Alkyl oder CO-N($C_1$-$C_4$-Alkyl)$_2$.

In besonders bevorzugter Weise bedeuten die Substituenten $R^1$, $R^2$ und $R^3$ unabhängig voneinander Wasserstoff, Chlor, Brom, Methyl, Ethyl, Methoxy oder Ethoxy.

Das gegebenenfalls substituierte $\alpha$-Naphthylisocyanat wird in der Friedel-Crafts-Umsetzung in beispielsweise 5-15 gew.-%iger, bevorzugt 8-12 gew.-%iger Lösung in einem der obengenannten Lösungsmittel in Gegenwart einer der obengenannten Katalysatoren umgesetzt. Beispielsweise kann es als 20-50 gew.-%ige Lösung zu vorgelegtem restlichen Lösungsmittel und vorgelegtem Katalysator zudosiert werden.

Die Friedel-Crafts-Reaktion wird beispielsweise bei einer Temperatur von 130-180°C, bevorzugt 150-165°C bis zur Beendigung durchgeführt. Das Ende der Reaktion kann in einfacher Weise durch analytische Verfolgung wie oben beschrieben, erkannt werden.

Von den oben erwähnten Friedel-Crafts-Katalysatoren wird in bevorzugter Weise $AlCl_3$ und/oder $AlBr_3$ in einer Menge von 1,2-3 Mol, bevorzugt 1,5-2,5 Mol pro Mol

des Isocyanats eingesetzt. Das gegebenenfalls substituierte δ-Naphthylisocyanat der Formel (I) wird hierbei zum gegebenenfalls substituierten Naphthostyril der Formel

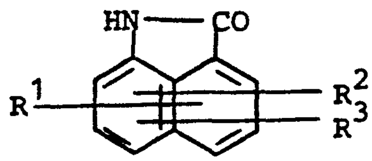

(II)

cyclisiert, worin

$R^1$, $R^2$ und $R^3$ den obengenannten Bedeutungsumfang haben.

Nach beendeter Cyclisierung wird das Reaktionsgemisch in der oben beschriebenen Weise, bevorzugt bei 50-200°C, mit einem kristallinen und wasserfreien Halogenid der Alkali- oder Erdalkalimetalle oder des gegebenenfalls substituierten Ammoniums, wie oben definiert, versetzt und gegebenenfalls nach einer Nachrührzeit durch destillative Wiedergewinnung des Lösungsmittels weiter verarbeitet. Hierbei kann in der beschriebenen Weise so verfahren werden, daß das Lösungsmittel teilweise oder vollständig vom Reaktionsansatz in direkt wiederverwendbarer Form abdestilliert wird. Beispielsweise wird bei einer Menge von 1,2-3 Mol $AlCl_3$ und/oder $AlBr_3$ für die Cyclisierung ein Halogenid des Natriums, Kaliums, Calciums oder des gegebenenfalls substituierten Ammoniums in einer mindestens äquimolaren Menge im Bereich von 1,2-3,5 Mol eingesetzt.

Die weitere Aufarbeitung des nach der destillativen

Gewinnung von mindestens einem Teil des Lösungsmittels verbleibenden Reaktionsgemisches geschieht ebenfalls in der oben beschriebenen Weise. So kann beispielsweise im Falle einer nicht vollständigen Wiedergewinnung des Lösungsmittels die erhaltene fließfähige Mischung aus Naphthostyril, restlichem Lösungsmittel und den anorganischen Stoffen auf Wasser gegeben werden, das gegebenenfalls Säure enthält, wobei sich eine wäßrige saure Salzlösung und eine Lösung von Naphthostyril im restlichen verwendeten Lösungsmittel bildet. Diese beiden Phasen können in einfacher Weise getrennt werden; arbeitet man im Bereich von etwa 10-20 gew.-%igen Naphthostyril-Lösungen, geschieht die Phasentrennung vorteilhaft in der Wärme, beispielsweise bei 40-110°C. Aus der abgetrennten organischen Phase kann sodann durch Kristallisation und Filtration ein kristallines Naphthostyril in hohen Ausbeuten isoliert werden. Die durch Phasentrennung erhaltene etwa 10-20 gew.-%ige Lösung des Naphthostyrils kann jedoch auch ohne Isolierung des Feststoffs direkt weiter verarbeitet werden, beispielsweise durch Alkylierung zum N-Ethylbenz-[c,d]-indol-2-on (N-Ethyl-naphtholactam). Selbstverständlich können für die Reinigung und Weiterverarbeitung solcher Lösungen bekannte Schritte, wie Klärfiltration, Konzentrierungs- oder Verdünnungsschritte angeschlossen werden.

In der oben beschriebenen Weise kann jedoch auch die erfindungsgemäße Lösungsmittel-Wiedergewinnung soweit fortgeführt werden, daß ein pulvriger Destillationsrückstand entsteht, der gegebenenfalls in angesäuertem

Wasser eingetragen wird. Hierbei lösen sich die anorganischen Stoffe, und das rohe Naphthostyril kann durch Filtration, Abpressen, Dekantieren oder Abschleudern isoliert werden, wobei es in besonders guter Ausbeute anfällt. Das so erhaltene rohe Naphthostyril ist in vielen Fällen direkt zur Weiterverarbeitung, beispielsweise zur Alkylierung zum N-Ethyl-naphtholactam geeignet. Selbstverständlich kann es jedoch, wenn eine höhere Reinheit benötigt wird, in bekannter Weise, z.B. durch Umkristallisation oder durch Umlösen (Auflösen in wäßrigem Alkali, gegebenenfalls Klärfiltration und anschließendes Ausfällen durch Säure) gereinigt werden. Auch die Sublimation im Vakuum kommt als Reinigungsoperation in Frage.

Durch die hier aufgezeigte Arbeitsweise kann die Aufarbeitung von Friedel-Crafts-Reaktionsgemischen in besseren Raum-Zeit-Ausbeuten als bisher durchgeführt werden. Am Beispiel der Cyclisierung von $\alpha$-Naphthylisocyanat zum Benz-/c,d/-indol-2-on kann so vorteilhaft bei der Cyclisierung in mäßig konzentrierter Lösung gearbeitet werden und anschließend in vorteilhafter Weise in konzentrierter Lösung aufgearbeitet werden. Außerdem fällt das wiedergewonnene Lösemittel einfach in wasserfreier Form an und kann direkt wiederverwendet werden.

Beispiel 1

In einem 7 ltr-Laborschaufeltrockner wurden in 4260 g (3270 ml) o-Dichlorbenzol 720 g (5,4 mol) $AlCl_3$ wasserfrei gelöst und auf 160°C erhitzt. Dazu tropfte man die Lösung von 360 g (2,13 mol) $\alpha$-Naphthylisocyanat in 1080 g (830 ml) o-Dichlorbenzol bei 160°C in 1 h zu und rührte die Mischung 1 h bei 160°C nach. Dann gab man bei 150-160°C 350 g (6 mol) Natriumchlorid in einer Portion zu, rührte 15 Minuten bei 160°C nach und kühlte auf 100°C. Dann wurde bei 90-100°C/35 mbar das gesamte Lösungsmittel abdestilliert. Man erhielt 5124 g (3933 ml) o-Dichlorbenzol mit einem Gehalt von $<$ 20 ppm Al als farblose Flüssigkeit zurück. Wiedergewinnungsrate 93 % vom Einsatz. Als Sumpfprodukt erhielt man 1545 g hellbraunes Pulver, das in 4000 g (4000 ml) Wasser eingetragen wurde. Man rührte 1 h bei 90°C nach und filtrierte das rohe Naphthostyril ab, wusch 2 mal mit je 2000 ml Wasser und trocknete das Produkt. Man erhielt 390 g 76 %iges Naphthostyril.
Ausbeute: 82,5 % der theoretischen Ausbeute

Beispiel 2

In einem 7 ltr-Laborschaufeltrockner wurden in 3450 g (2650 ml) o-Dichlorbenzol 708 g (5,32 mol) $AlCl_3$ wasserfrei gelöst und auf 160°C erhitzt. Dazu tropfte man die Lösung von 600 g (3,55 mol) $\alpha$-Naphthylisocyanat in 1800 g (1380 ml) o-Dichlorbenzol bei 160°C in 1 h zu und

rührte die Mischung 30 Minuten bei 160°C nach. Dann gab man bei 150-160°C 321 g (5,5 mol) Natriumchlorid in einer Portion zu, rührte 15 Minuten bei 160°C nach und kühlte auf 100°C ab. Man destillierte das Lösemittel bei 100°/12 mbar ab und erhält 91 % vom Einsatz Dichlorbenzol. Man hydrolysierte den Rückstand wie in Beispiel 1 angegeben. Man erhielt 692 g 65 %iges Naphthostyril. Ausbeute: 75 % der theoretischen Ausbeute

Beispiel 3

In einem 2-ltr-4-Halskolben wurden 522 g Dichlorbenzol-Isomerengemisch mit 85 % ortho- und 15 % para-Dichlorbenzol vorgelegt und 94,4 g (0,71 mol) Aluminiumchlorid eingetragen. Die Mischung wurde auf 150°C aufgeheizt und die Lösung von 60 g (0,355 mol) α-Naphthylisocyanat in 180 g Dichlorbenzol-Isomerengemisch in 1 h zugetropft. Dabei wurde die Temperatur auf 160°C erhöht und 1 h bei 160°C nachgerührt. Man kühlte auf 100°C, gab 46 g (0,80 mol) Natriumchlorid zu und rührte 1 h bei 100°C nach. Dann wurden bei 100°C/10 mbar 495 g Dichlorbenzol (70 % von Einsatz) abdestilliert und der 100°C warme Sumpf auf 550 g Wasser ausgetragen. Der Kolben wurde mit 20 ml Dichlorbenzol nachgewaschen. Die Hydrolysemischung wurde auf 90-95°C erwärmt und bei stillgesetztem Rührer die untere organische Phase bei 98°C abgelassen. Man gab noch 20 ml Dichlorbenzol zur Wasserphase, verrührte kurz und trennte die restliche organische Phase ab.

Man erhielt 280,5 g einer 16,8 %igen Lösung von Naphthostyril in Dichlorbenzol, die direkt in die Herstellung von N-Ethyl-naphtholactam eingesetzt werden kann. Ausbeute: 78,4 % der theoretischen Ausbeute

Beispiel 4

Zur Lösung von 266 g (2,0 mol) Aluminiumchlorid in 1240 g (954 ml) o-Dichlorbenzol wurde bei 160°C die Lösung von 169 g (1 mol) α-Naphthylisocyanat in 507 g (390 ml) o-Dichlorbenzol in 1 h zugetropft und die Reaktionsmischung 1 h bei 160°C nachgerührt. Man kühlte auf 130°C und gab 130 g (2,22 mol) Natriumchlorid zu, rührte 30 Minuten bei 130°C nach und destillierte bei 130°C im Sumpf 783 g (602 ml) Dichlorbenzol (44,8 % vom Einsatz) ab. Man goß den Sumpf in 1550 ml Wasser und trennte bei 95°C die untere organische Phase ab. Man gab noch 20 ml Dichlorbenzol in die Wasserphase, verrührte kurz und trennte die organische Phase unten ab. Die organische Phase wurde kalt gerührt und bei 20°C abgesaugt. Der Filterkuchen wurde mit 50 ml Dichlorbenzol gewaschen und getrocknet. Man erhielt 158,4 g 87,5 %iges Naphthostyril.
Ausbeute: 82 % der theoretischen Ausbeute

Beispiel 5

Zur Lösung von 70,8 g (0,53 mol) Aluminiumchlorid in 345 g o-Dichlorbenzol wurde bei 155°C die Lösung von

60 g (0,355 mol) α-Naphthylisocyanat in 180 g o-Dichlorbenzol zugetropft. Die Temperatur wurde dabei in 1 h auf 160°C angehoben und 30 Minuten bei 160°C nachgerührt. Dann gab man bei 150°C 32,1 g (0,55 mol) Natriumchlorid zu, rührte noch 15 Minuten bei 150°C und kühlte auf 100°C ab. Dann wurden bei 76°C/24 mbar 324 g Dichlorbenzol (61,7 % vom Einsatz) abdestilliert. Der flüssige Sumpf wurde in 550 g Wasser gegossen und der Kolben mit 50 ml o-Dichlorbenzol und 50 ml Wasser gespült. Die Waschlösungen wurden zum Sumpf gegeben und das o-Dichlorbenzol durch Einblasen von Wasserdampf abdestilliert. Die verbleibende Wasserphase wurde gekühlt und das rohe Naphthostyril als linsenförmige Partikel durch Filtrieren isoliert. Nach Waschen mit 100 ml Wasser und Trocknung erhielt man 68 g 72,3 %iges Naphthostyril mit Fp: 161°C.
Ausbeute: 82 % der theoretischen Ausbeute.

Beispiel 6 bis 15

Die in der Tabelle dargestellten Ergebnisse zeigen, welche Ausbeuten man erhält, wenn man nach der Vorschrift des Beispiel 5 arbeitet, aber andere Metallhalogenide vor der Destillation des Lösemittels zusetzt:

| Nr. | Metallhalogenid | Mol Metallhalogenid pro Mol $AlCl_3$ | Ausbeute an Naphthostyril (%) | Wiedergewinnungsrate des Lösemittels (% vom Einsatz) |
|---|---|---|---|---|
| 6 | NaBr | 1,2 | 84 | 69 |
| 7 | KCl | 1,1 | 82 | 80 |
| 8 | $MgCl_2$ | 1,1 | 80 | 64 |
| 9 | $CaCl_2$ | 0,6 | 80 | 69 |
| 10 | $CaCl_2$ | 1,1 | 82 | 79 |
| 11 | $NH_4Cl$ | 1,3 | 82 | 79 |
| 12 | KBr | 1,2 | 83 | 79 |
| 13 | NaCl + KCl | 0,6 + 0,5 | 82 | 80 |
| 14 | LiCl | 1,1 | 80 | 75 |
| 15 | KF | 1,1 | 80 | 78 |

Patentansprüche

1. Verfahren zur Durchführung von Friedel-Crafts-Reaktionen und Friedel-Crafts-ähnlichen Reaktionen in inerten wasserfreien Lösungsmitteln und in Gegenwart von wasserfreien Halogeniden des Aluminiums, Eisen oder Zinks, dadurch gekennzeichnet, daß man nach Beendigung der Reaktion dem Reaktionsgemisch Halogenide der Alkali- oder Erdalkalimetalle oder des gegebenenfalls substituierten Ammoniums in einer Menge von 100-150 % der zur Bildung der Tetrahalogenosalze des Aluminiums, Eisens oder Zinks erforderlichen Menge bei 20-250°C zusetzt und gegebenenfalls nach einer Nachreaktionszeit das Lösungsmittel mindestens teilweise in wasserfreier Form durch Destillation aus dem Reaktionsgemisch zurückgewinnt.

2. Verfahren nach Anspruch 1, dadurch gekennzeichnet, daß man Reaktionsgemische, die mit Halogeniden des Aluminiums und Eisens erhalten werden, einsetzt.

3. Verfahren nach Anspruch 1, dadurch gekennzeichnet, daß man Reaktionsgemische, die mit $AlCl_3$ und/oder $AlBr_3$ erhalten wurden, einsetzt.

4. Verfahren nach Anspruch 1 bis 3, dadurch gekennzeichnet, daß man Halogenide von Natrium, Kalium, Calcium oder gegebenenfalls substituiertem Ammonium zusetzt.

0167821

5. Verfahren nach Anspruch 1 bis 3, dadurch gekennzeichnet, daß man Halogenide von Natrium oder Kalium zusetzt.

6. Verfahren nach Anspruch 1 bis 5, dadurch gekennzeichnet, daß man 100-120 % der zur Bildung der Tetrahalogenosalze erforderlichen Menge der Halogenide der Alkali- oder Erdalkalimetalle oder des gegebenenfalls substituierten Ammoniums zusetzt.

7. Verfahren nach Anspruch 1 bis 6, dadurch gekennzeichnet, daß man aus dem Reaktionsgemisch das Lösungsmittel vollständig abdestilliert und gegebenenfalls anschließend, gegebenenfalls unter vermindertem Druck, das Produkt destillativ gewinnt.

8. Verfahren zur Herstellung von gegebenenfalls substituierten Benz-[c,d]-indol-2-on (Naphthostyril) durch Umsetzung von gegebenenfalls substituiertem α-Naphthylisocyanat in inerten wasserfreien Lösungsmitteln in Gegenwart von wasserfreien Halogeniden des Aluminiums, Eisens oder Zinks, dadurch gekennzeichnet, daß man nach Beendigung der Reaktion nach Anspruch 1 bis 7 weiter verfährt.

9. Verfahren nach Anspruch 8, dadurch gekennzeichnet, daß die Cyclisierung bei 130-180°C und der Zusatz von Halogeniden des Natriums, Kaliums, Calciums oder des gegebenenfalls substituierten Ammoniums bei 50-200°C erfolgt.

10. Verfahren nach Anspruch 8 und 9, dadurch gekennzeichnet, daß $AlCl_3$ und/oder $AlBr_3$ in einer Menge von 1,2-3 Mol pro Mol α-Naphthylisocyanat und das Halogenid in mindestens äquimolarer Menge zum $AlCl_3$ und/oder $AlBr_3$ im Bereich von 1,2-3,5 Mol eingesetzt werden.

Europäisches Patentamt

# EUROPÄISCHER RECHERCHENBERICHT

0167821
Nummer der Anmeldung

EP 85 10 6909

| Kategorie | EINSCHLÄGIGE DOKUMENTE<br>Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| X | US-A-3 275 699 (D.G. WALKER et al.)<br>* Insgesamt * | 1-7 | C 07 D 209/92 |
| Y | | 8-10 | |
| | --- | | |
| Y | DE-A-2 635 693 (BAYER AG)<br>* Seite 4, Zeile 20 - Seite 6, Zeile 19 * | 8-10 | |
| | --- | | |
| A | CHEMICAL ABSTRACTS, Band 48, Nr. 8, 25. April 1954, Abstract Nr. 4487 d-g, Columbus, Ohio, US; N.S. DOKUNIKHIN et al.: "Organic isocyanates. II. Transformation of isocyanates of the naphthalenes series under the action of aluminium chloride", & ZHUR. OBSHCHEI KHIM. 1953, 23, 798-800 | 1,8-10 | RECHERCHIERTE SACHGEBIETE (Int. Cl.4)<br>C 07 D 209/00 |
| | --- | | |
| A | DE-C- 887 198 (AMERICAN CYANAMID CO.)<br>* Ansprüche * | 8-10 | |
| | --- | | |
| A,D | DE-A-2 700 649 (BAYER AG)<br>* Ansprüche * | 8-10 | |
| | ----- | | |

Der vorliegende Recherchenbericht wurde für alle Patentansprüche erstellt.

| Recherchenort | Abschlußdatum der Recherche | Prüfer |
|---|---|---|
| BERLIN | 04-09-1985 | VAN AMSTERDAM L.J.P. |

KATEGORIE DER GENANNTEN DOKUMENTEN

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503. 03.82